# EUROPEAN PATENT SPECIFICATION

(11) **EP 2 897 924 B1**
(45) Date of publication and mention of the grant of the patent: **14.11.2018**
(21) Application number: 13774218.5
(22) Date of filing: 13.09.2013
(51) Int. Cl.: C05F 11/00, C05F 17/00, C05F 7/02, C05D 9/00, C02F 11/04, C02F 103/28, C12P 5/02, C12P 7/08

(54) **A METHOD OF PROCESSING SIDE FLOWS AND WASTE FLOWS OF PULP AND PAPER INDUSTRY AND A FERTILIZER**
VERFAHREN ZUR VERARBEITUNG VON NEBENSTRÖMEN UND ABFALLSTRÖMEN IN DER PULPE- UND PAPIERINDUSTRIE SOWIE DÜNGEMITTEL
PROCÉDÉ DE TRAITEMENT DE FLUX DE MATIÈRES RECYCLABLES ET DE FLUX DE DÉCHETS DE L'INDUSTRIE PAPETIÈRE ET ENGRAIS

(30) Priority: 18.09.2012 FI 20125960
(43) Date of publication of application: 29.07.2015
(73) Proprietor: BioA Oy, 48600 Kotka (FI)
(72) Inventor: JÄRVINEN, Jari, 45160 Kouvola (FI)
(74) Representative: Genip Oy
(86) International application number: PCT/FI2013/050890
(87) International publication number: WO 2014/044905

(56) References cited:
- WO-A2-2011/151511
- US-A1- 2009 095 673

## Description

### Technical field

The present invention relates to a method of processing various side flows and waste flows of pulp and paper industry in the manner described in the preamble of claim 1 and a fertilizer described in the preamble of claim 14.

### Background art

In the following description the term 'side flow' is understood as such a material flow from, for instance, an industrial facility that the industrial facility cannot any more use in its own processes but that may be taken forward to be utilized by another user. A "waste flow" is here understood as a flow from an industrial facility that neither the industrial facility itself not any other facility is able to utilize, i.e. a traditionally worthless flow.

A feature common to historical prior art methods of handling waste and, unfortunately also side flows, irrespective of their municipal or industrial origin, has been that the main goal has just been to get rid of the waste or side flows with as low expenses as possible. Dumping the waste and side flows to landfill has earlier been a popular way. Lately, for preventing harmful substances from getting into the ground waste incineration has been taken into use. Waste incineration is very often performed at a very low efficiency and, moreover, in such a way that combustion gases are allowed to be discharged into the atmosphere in a way that increases environmental load in the form of either only carbon dioxide or possibly many other compounds, in some cases even in the form of toxic or almost toxic compounds. Incineration of the waste leads also to, in practice, final loss of nutrients, as combusting the waste or side flows normally means that, for instance, the phosphorus from the flows remains in the ash that contains heavy metals to such an extent that the ash cannot be used but only as landfill in such a manner that plants cannot utilize the phosphorus any more.

In recent years the strengthening legislation has led to more and more efficient ways of handling of both municipal and industrial waste and side flows. For instance, in some advanced cases, a certain waste or side flow is taken, for example, to a bio ethanol plant, where specifically bio ethanol is sought to be recovered from the waste, the rest of the end product ending up as waste, i.e. to be either incinerated, handled in connection with waste water processes or dumped as landfill. In some cases also the residual matter from the primary use finds some other application. For example, if the raw material is clean bakery waste, the residual from an ethanol plant may be further used as livestock fodder. However, if the raw material is some even slightly less pure ethanol raw material, the residual from ethanol production processes has been traditionally taken as waste slurry to municipal waste processing.

US-A1-20090095673 discusses systems and methods provided for converting organic waste materials from a municipal waste stream to useful products, such as fuels. Through the use of a biomixer and a hydropulper, as well as through sorting and screening, the organic waste materials are progressively reduced in size and cleaned of contamination. The resulting uniform biomass is suitable for anaerobic digestion to produce biogas and a residual solid that is suitable for producing a high quality compost. A quantity of liquid organic waste material can be added to the biomixer, to the hydropulper and/or to the anaerobic digester. The quantity of liquid organic waste material can be obtained by separating the liquids from any containers and can be stored in a holding tank prior to being added to the biomixer, the hydropulper and/or the anaerobic digester.

In recent years a few further patent documents have come up discussing a more comprehensive approach for processing organic waste material. As examples of those documents WO-A2-2011151511, WO-A2-2009090476, US-A1-2008135474, and US-A1-2008050800 may be mentioned, the disclosures of which are fully incorporated herein by reference.

The documents teach how organic waste material received from various sources, both agricultural, industrial and community sources, are collected and introduced in a preferred order into a so called bio refinery comprising a number of different process units, like for instance one or more of ethanol plant (fermentation), anaerobic digester (biogas production), and algae reactor. Sometimes also a biodiesel plant, and/or a power plant may be considered to belong to the bio refinery, if it/they is/are using directly the end products of the above listed process units.

The algae reactor receives, in addition to the raw material received from the ethanol plant, carbon dioxide, water and nutrients from the anaerobic digester and flue gas from the power plant. The algae solids may be taken, in addition to the ethanol plant/fermentation, also to the anaerobic digester/biogas production, the power plant and the livestock facility. The algae reactor may also produce algae oil that may be used at the biodiesel plant. The residual mass from the bio refinery, i.e. so called digestate, may be utilized as a fertilizer when combined with ash or some other water absorbing material from an appropriate source.

The anaerobic digester receives waste or side flows from suitable sources, and produces, in addition to the already listed products, ammonia, biogas and/or methane for the power plant, and biogas and/or methane for the biodiesel plant.

The power plant is designed to produce energy that may be taken out of the process, if desired, in the form of district heating or electricity for instance. However, a significant amount of energy fed in the power plant in the form of organic material is needed for various heating purposes in the anaerobic digester, the algae reactor and the biodiesel plant, i.e. within the process itself. The ammonia produced by the anaerobic digester and fed to the power plant is utilized in scrubbing sulphur from the flue gas or as an additive in the fertilizer production.

Thus the process, which is very complicated and includes many different technologies and process units, produces ethanol, electricity and biodiesel that can be sold further. In addition to the already discussed products, the kind of processes discussed above also produces rejectable organic material that may be used as a fertilizer. Very often the solids left over after the treatment at the ethanol plant, i.e. fermentation, the anaerobic digester and/or the algae reactor, if not, for some reason, capable of being used as feedstock for livestock facility, are either combusted or used as a fertilizer. However, a significant problem in the use of the left-over solids as fuel or fertilizer is their high water content. In both cases the left-over solids must, in accordance with the practices of prior art, be dewatered and/or dried, which consumes a significant amount of energy. In fact, the amount of energy spent in dewatering and/or drying is in many cases comparable to the total energy content of left-over solids.

As to further prior art teachings it should be understood that some prior art documents teach how nitrogen is stripped, or by some other way produced, from ammonia and introduced into the organic solids discharged from the waste treatment facility as fertilizer. Stripping means a simple process where ammonia from the air is scrubbed with sulphuric acid and recovered as a 40% TS (total solids, dry matter) ammonium sulphate solution. Ammonium sulphate is utilized as a fertilizer and/or for soil enrichment production.

Thus, in spite of the fact that, for instance, the above discussed documents teach a proper way of handling various municipal, industrial and agricultural waste and side flows, such waste treatment facilities have not found any broader acceptance on the market. One of the main reasons is the economical aspect of the waste treatment facility. As taught by the cited documents, a comprehensive waste treatment facility requires a number of different treatment methods and processes having equipment of their own. However, a fact is that the equipment requires complex instrumentation and control of the processes. The waste and side flows have to be brought to the waste treatment facility. And the waste and side flows brought to the site have to be chosen correctly. Thus, both the investments in building and costs of running the waste treatment facility are substantially high compared to the value of the end products normally collected from the waste treatment facility.

A good example of this may be found in pulp and paper industry where it is common practice to incinerate all side and waste flows. A pulp and paper mill creates side and waste flows in many phases of its operation. Firstly, when the wood is taken into use at the wood yard, the logs may be sawed into shorter particles, then they are debarked and possibly chipped, which results in not only bark but also sawdust and other wood particles that has to be got rid of. Secondly, when the mechanical, chemi-mechanical or chemical pulp leaves the various screening or sorting stages, a reject flow including particles that have not passed the screens is removed from the mill. Also in later stages of pulp and paper mill various flows, for instance filtrates containing a small but still significant amount of fibers, fines, fillers, various treatment chemicals etc. are rejected. It could also be said that the farther the rejectable material gets into the processes the more water the rejected flow contains and the more chemicals and other material of non-wood origin may be found in the reject flow.

All these material flows, i.e. bark, sawdust and other wood particles from the woodyard and rejected side and waste flows have been traditionally combined, and taken to a bark boiler, the main purpose of which is to turn the combustible material into energy. It has to be understood that bark, sawdust and other wood particles, sometimes called as hog fuel, from the woodyard are, by their nature, as they cannot be utilized in any better manner, a good source of energy. Therefore they are combusted in a so called bark boiler. However, very often the settled slurry of the biological wastewater treatment facility of a pulp or a paper mill is taken to a bark boiler for combustion. The settled slurry contains as much solids separated by settling from the mill's wastewaters as possible, as the clarified effluent water from the biological facility is normally dumped to rivers, lakes, seas or oceans. In other words, the settled bio slurry contains both organic and inorganic matter. A considerable problem when delivering the settled bio slurry to the bark boiler for incineration is its high water content, and especially its weak dewaterability. In spite of the fact that the slurry is dewatered, its water content may still be so high that it cannot be introduced into the bark boiler. Therefore a part or all of the fibrous slurry separated by the primary clarifier from the mill's filtrates prior to the biological wastewater treatment facility is introduced in higher consistency in the bio slurry to increase its dry matter content to combustible level. Sometimes also peat is used for the same purpose. In other words, the bark boiler is used, not only to recover energy from the bark and other wood based materials, but to get rid of various problematic waste materials. It has not been considered a worthwhile problem that huge amounts of water has to be evaporated until the waste and side flows are in combustible dry matter content.

Only recently it has been discussed, for instance in WO-A2-2011151511, that the waste and side flows could be utilized more efficiently so that not only energy but also various other valuable end products like ethanol, methanol and possibly also fertilizer could be processed from municipal and industrial waste and side flows. This has also evolved an idea that possibly also the ash recovered from the bark boiler could be used in fertilizer production. However, when running experiments on the bio refinery built in connection with a pulp mill and utilizing the main ideas discussed in the WO-A2-2011151511 several problems were encountered.

Firstly, when studying the ash content of a bark boiler where in addition to ash also bio slurries and fibrous slurries were incinerated it was learned that the ash was very fine and contained heavy metals to such a degree that the ash itself could not be utilized in the production of fertilizer. In other words, ash contains heavy metals (Cd, Cu, Cr, Pb, Ni, Zn, As, V) that originate from the combusted fuel. The national or community legislation limits the contents of heavy metals in the fertilizers. In practice, if the cadmium/phosphorus ratio in the residual slurry - ash mixture is low enough the mixture may be used as the fertilizer. The ordinary heavy metal content in ash results normally in too high a concentration of heavy metals in the fertilizer, whereby heavy metals should be removed from the ash. Article "Theoretical Feasibility for Ecological Biomass Ash Recirculation: Chemical Equilibrium Behavior of Nutrient Elements and Heavy Metals during Combustion" in Environmental Science & Technology, vol. 31, No. 9, 1997 teaches that heavy metals adhere to small and light ash particles in connection with high temperature combustion and, based on that behavior may be separated from fly ash by means of a hot cyclone. Thereby, prior art teaches an affordable way of treating fly ash so that the heavy metal content therein is reduced to such a level that when added to the fertilizer, or actually to the residual slurry of the ethanol or biogas production, the heavy metal content in the fertilizer remains in acceptable level.

However, by treating the ash by means of pneumatic classifying a part, approximately one quarter, of the ash was recovered in such fineness that it could be utilized in construction material industry, for instance in place of cement in the manufacture of concrete. The ash cannot be used to replace the cement entirely but roughly up to about one third of the cement. Another thing that was also learned was that the pneumatic classifying did not help in separating heavy metals from the ash or even in getting any considerable difference between the heavy metal content in a fine ash fraction and that in a coarse ash fraction. Thus it was an easy conclusion that the ash from a bark boiler could not be used in fertilizer production.

Secondly, it was learned at the bio refinery that the growth rate of the microbe cultivation in a fermentation reactor was not nearly to what was expected. Thus the ethanol production was also far from expected. It was concluded that the microbes in the fermentation reactor either could not utilize the fibrous slurries introduced into the fermentation reactor, or the flows included something that prevented the efficient growth of the microbe cultivation.

Thirdly, the production of fertilizer is an attractive choice for handling the solid residues of a bio refinery, as fertilizer, and especially organic fertilizer, is a valuable product in several aspects. First of all it is a product that may be sold with profit whereby its production is economically justifiable. Secondly, the solid residue of a bio refinery contains nutrients (phosphorus, nitrogen, potassium) that plants may utilize, and if the solid residue is incinerated or dumped as landfill (in the manner done in prior art processes) the nutrients are lost. The national or community legislation concerning fertilizers divides the fertilizers into two groups, i.e. organic fertilizers or bio fertilizers and ordinary or non-organic fertilizers of which only organic fertilizers may be used in organic food production. Further the legislation may either directly or indirectly dictate, for instance, what kind of raw materials may be used in the production of organic and non-organic fertilizers, how the fertilizers have to be manufactured, and what kind of impurities are allowed in the fertilizer and the allowed amount of such impurities. Naturally, the waste materials that may be used in the production of organic fertilizers are bound by stricter requirements than waste materials used in the production of non-organic fertilizers. Now that there is a high demand for organic fertilizers on the market, it makes sense to aim at organic fertilizer production, and use only such waste and side flows for non-organic fertilizer production that cannot be used for organic fertilizer production. Now, for instance, the legislation rules that a fertilizer that may be called organic fertilizer must be manufactured such that all the raw materials are recovered or collected from the same site. Thus, the legislation solves the problems relating to raw material transport, but brings about a new problem, i.e. finding an industrial site or plant where all the required raw materials are available.

### Brief summary of the Invention

Thus, an object of the present invention is to raise the state of the art in the area of recycling waste and side flows of pulp and paper industry by introducing a method capable of minimizing at least some problems and drawbacks of prior art.

Another more detailed object of the present invention is to be able to collect the waste and side flows from a single industrial facility whereby no transportation is needed and the requirements of the legislation concerning organic fertilizer production are fulfilled.

Still another more detailed object of the present invention is to find a way to treat the bark, sawdust, wood particles, and fibrous slurries and/or bio slurries of pulp and paper industry such that each one of them is processed in the most economical manner, i.e. affordable processes resulting in high value end products.

Yet another more detailed object of the present invention is to find a way to treat the ashes recovered from the incineration of pulp and paper industry such that the ashes may be accepted in at least one of construction material industry and fertilizer production.

A further more detailed object of the present invention is to find a reason or cause why the fiber slurries hamper the operation of the ethanol fermentation reactor, remove the cause and thus to raise the production of ethanol in a considerably higher level.

At least some of the above and other objects of the present invention are reached by a method of processing waste and side flows of pulp and paper industry, the waste and side flows comprising at least bark and other wood based waste flows, so called hog fuel, and at least one fibrous waste flow; the processing arrangement of a pulp and/or a paper mill comprising at least a bark boiler for combusting hog fuel, a biological wastewater treatment plant and a bio refinery comprising at least fermentation/ethanol production, the method comprising the steps of introducing hog fuel to the bark boiler for producing ash, introducing the at least one fibrous waste flow to a treatment device for dividing the at least one fibrous waste flow to a fibrous primary slurry and a first effluent, introducing the first effluent to the biological wastewater treatment plant, and in which method
- the processing arrangement is provided with a separator unit,
- the fibrous primary slurry is introduced into the separator unit,
- the fibrous primary slurry is divided by means of the separator unit into at least two fractions, i.e. a light mainly organic fraction and at least one heavy mainly inorganic fraction,
- the light mainly organic fraction is introduced to the bio refinery for producing at least ethanol and digestate,
- the digestate is taken from the bio refinery to fertilizer production,
- hog fuel is introduced to the bark boiler for producing ash,
- the ash is taken from the bark boiler to fractionation,
- the ash is fractionated into at least one coarse fraction lean in heavy metals and a fine ash fraction rich in heavy metals, and
- the at least one coarse ash fraction lean in heavy metals is taken to fertilizer production for producing a fertilizer mixture together with the digestate.

At least some of the above and other objects of the present invention are reached by a fertilizer produced such that the fertilizer is formed, at least partially, of the digestate collected from a bio refinery treating one or more fibrous waste flows of a pulp and/or paper mill from which fibrous waste flows heavy mainly inorganic fraction is separated and of an ash fraction lean in heavy metals collected from a bark boiler of a pulp and/or paper mill.

Other characteristic features of the present invention become evident from the appended dependent claims and the following description of the embodiments of the present invention.

By applying the present invention at least some of the following advantages are gained:
- binding of nitrogen and phosphorus to algae and further on in fertilizer
- not requiring chemical processing
- facilitated fulfilling of emission standards
- instead of incinerating the waste and side flows, utilizes the flows efficiently
- soil depletion is prevented by recovering, among others, phosphorus into a biofertilizer, which reduces the need for chemical fertilizers
- nutrient cycle becomes more effective (for example, one is able to recover more phosphorus for reuse)
- the amount of waste for final disposal is reduced
- total energy produced in the process is greater than the energy from separate processes
- the amount of waste water leaving the process is decreased, because ash binds moisture into the fertilizer
- organic fertilizer including phosphorus and nitrogen may be sold for organic farming
- ash replaces the potassium as soil enhancing agent
- spreading both the fertilizer and the soil enhancing agent simultaneously reduces work at farms and the compaction of the soil.

### Brief Description of Drawing

In the following, the prior art and the present invention is discussed in more detail by referring to the appended drawings, of which
Figure 1 illustrates schematically an optional prior art treatment of various waste and side flows of a pulp and paper industry,
Figure 2 illustrates schematically another optional prior art treatment of various waste and side flows of a pulp and paper industry,
Figure 3 illustrates schematically a novel way of treating various pulp and paper industry waste and side flows in accordance with a preferred embodiment of the present invention, and
Figure 4 illustrates schematically the treatment of various pulp and paper industry waste and side flows in accordance with a preferred embodiment of the present invention.

### Detailed Description of Drawings

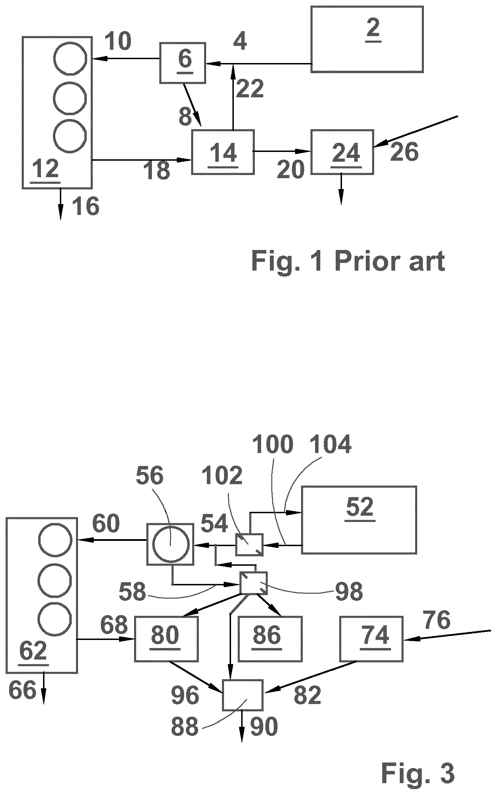

Figure 1 discusses schematically a prior art way of treating waste and side flows of pulp and paper industry (P&P industry). The pulp and/or paper mill 2 discharges various reject and filtrate flows 4 that contain solids including but not limited to at least a few of knots, sticks, fibers, fines, fillers, plastics, inks, sand, metal, glass, shives, and hot melts as well as various chemicals to a primary clarifier 6, which separates, normally by means of settling, a so called primary slurry 8 from the waste water 10 containing fine impurities. The reject and filtrate flow 4 contains, for instance, some acceptable fiber material for a reason discussed in more detail later in this description. The waste water 10 is taken to a secondary clarifier 12, which is normally a biological treatment plant or facility, where the aim is to convert the waste by microorganisms to carbon dioxide and water while consuming oxygen. The primary slurry 8 is taken to a dewatering unit 14, which may be a screw press, for example. The secondary clarifier 12 (i.e. the biological waste water treatment plant) produces clarified effluent 16, which is released to rivers, lakes, seas or oceans, and a secondary slurry 18, which is taken to the dewatering unit 14, too. In the dewatering unit 14 the primary slurry 8 and the secondary slurry 18 are dewatered such that final slurry 20 and water 22 are recovered. The recovered water 22 is taken to the feed of the primary clarifier 6 and the final slurry 20 to either landfill or for combustion. Since the landfill is not a popular way of disposing the slurries, a preferred choice has lately been to introduce the final slurry 20 to a bark boiler 24 together with hog fuel 26, i.e. bark and other wood based fuel. Sometimes also other waste wood that cannot be used for pulp, paper, tissue or board production and/or peat are added to the bark boiler 24.

The main reason why the primary and the secondary slurries 8 and 18 are combined is the high water content of the secondary slurry 18 and difficulties in dewatering the secondary slurry alone. As a result of the addition of the primary slurry 8 to the secondary slurry the solid material content, i.e. the consistency, of the final slurry 20 is raised to a significantly higher level than when dewatering secondary slurry alone. Especially when both the equipment and the energy spent in dewatering is taken into consideration. Thus the prior art waste water treatment aims at the incineration of these bio slurries. However, the incineration of the bio slurry is nothing but a way to get rid of the slurry. It means, for instance, that huge amount of water has to be evaporated until the slurry is combustible, and that all valuable minerals, nutrients, etc. in the slurry are lost for good, as they end up in the ash that contains heavy metals from the hog fuel. And, when the primary slurry is added to the bio slurry also a considerable amount of fiber and other material applicable in pulp, paper, tissue or board production is lost, too.

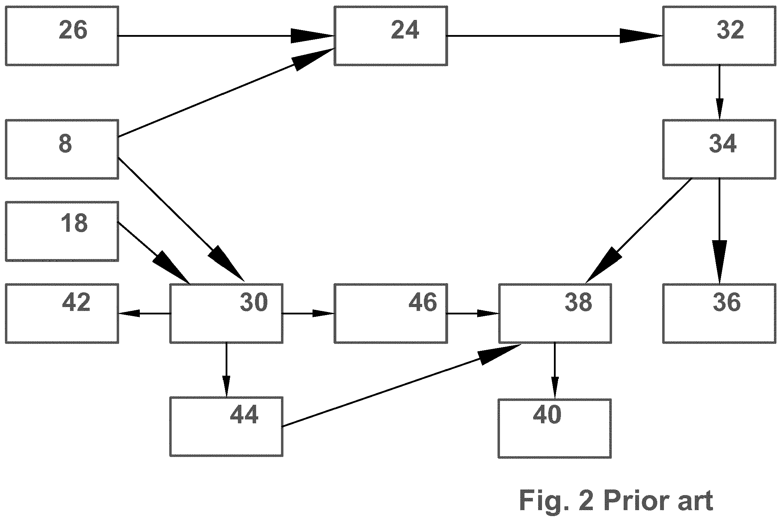

Figure 2 discusses schematically a few prior art treatment options for different side and waste flows of a pulp mill or a pulp and paper mill, i.e. of pulp and paper industry from slightly another perspective. Of the pulp mill processes only the bark boiler 24 and the bio refinery 30 has, in fact, been shown in Figure 2. The bio refinery includes one or more of at least one fermentation reactor (for ethanol production), at least one anaerobic digester (for biogas production), and at least one algae cultivation unit. The rest of the boxes in Figure 2 represent the raw materials, i.e. the side and waste flows, the processes, the intermediate products and the end products of the overall treatment processes. There are three raw materials, i.e. waste and side flows shown in Figure 2. The first one is bark, saw dust and other material 26, so called hog fuel, originating from debarking and other handling of logs at the wood yard. The second one is fibrous slurries 8 collected as residues from the various treatment stages of the pulp or paper mill, including rejects from various screening and sorting stages, including but not limited to chip screening, brown stock screening, fine screening, and cleaning, as well as waste fractions from delignification, bleaching, 0- water etc. These may also be recovered as the primary slurry from the primary clarifier, as discussed in Figure 1. And the third one is the bio slurries 18 from the pulp and/or paper mill's waste water treatment facility.

It is common practice to incinerate the bark and other wood material 26 from the wood yard in the bark boiler 24 the heat generated by the bark boiler being used for instance in steam generation. In addition to combusting bark and the wood material the boiler is normally used for combusting at least a part of the fibrous slurries 8, sometimes also at least a part of the bio slurries 18, too. However, in spite of the fact that the fibrous slurries 8 and bio slurries 18 are usually dewatered before feeding in the bark boiler 24 their water content is still so high that a considerable amount of the heat generated in the boiler 24 is, in a way, wasted in evaporating the water, i.e. drying the fibrous dewatered slurries in combustible condition.

The ash 32 resulting from the combustion in the bark boiler 24 contains heavy metals, which are mostly concentrated in or originating from the bark. The presence of heavy metals limits the use of boiler ash 32 a great deal. Traditionally the ash has been dumped, i.e. taken to landfill. Later it has been suggested that the ash could be fractionated such that the fine fraction could be used in construction material industry, for example replacing partly the cement in the production of concrete. The coarser fractions may still have ended up to landfill. A further suggestion has been to fractionate 34 the ash such that the heavy metals would be taken out in connection with one fraction 36 whereafter the other fraction could be used in the production 38 of a fertilizer 40. The latter option has been mentioned in WO-A2-2011151511.

The other process unit of the pulp mill or the pulp and paper mill shown in Figure 2, i.e. the bio refinery 30 has been discussed in more detail both above and in WO-A2-2011151511, whereby its operation and detailed structure is not disclosed here in any more detail. It has only been shown in Figure 2 that the bio refinery 30 uses as its raw material bio slurries 18 and at least a part of the fibrous slurries 8. They are treated in the bio refinery by means of at least one of fermentation, anaerobic digestion, and the use of algae such that at least one of ethanol and methanol 42, nitrous compound 44, for instance in the form of nitrogen sulphite, and residual slurry 46, so called digestate is recovered.

The ethanol and methanol 42 are valuable end products, which may be sold for further processing or combusted for creating heat, steam and/or electricity, for instance. The nitrous compound 44 may be introduced to the production 38 of the fertilizer 40. The residual slurry 46 from the bio refinery, so called digestate is normally dewatered by, for instance, centrifuging and then taken to be mixed with the ash fraction lean in heavy metals together with the nitrous compound 44. Thereafter the mixture may be further dewatered, and treated optionally in a still further dewatering step by means of a screw press, for reaching a high enough dry content for granulation and packing in sacks so that the fertilizer 40 may be sold further. The resulting fertilizer may be organic (biofertilizer) or non-organic fertilizer depending on the origin of its raw materials.

The above discussed prior art processes and procedures have a few problems, which will be discussed in more detail in the following.

Firstly when testing the process discussed in WO-A2-2011151511 in connection with a pulp mill the option of fractionating the bark boiler ash in a heavy metal rich fraction and a heavy metal lean fraction proved to be non-functional as the fractionation did not result in any significant variations in the heavy metal content between the various fractions. The ash that was used originally in the experiments was the result of incinerating both bark and other solids originating from the wood yard treatment of logs having a particle size around 25 mm and fibrous slurries having a particle size in the area of millimeters. However, as it was already known that evaporating excess water from the fibrous slurries is, by no means, economical the testing included also studying a process where the waste of energy in evaporation was avoided by feeding both fibrous slurries and bio slurries to the bio refinery. When this was done, the bark boiler received only bark and other wood yard based wood material. Now that, thereafter, the ash recovered from the boiler was studied, it was learned that the size distribution of the ash particulates was tripled or quadrupled compared with the ash of combined combustion of bark and fibrous slurries. Further when fractionating such ash, it was learned that the finest fraction was rich in heavy metals, and the other, coarser fraction/s was/were lean in heavy metals. The heavy metal content of the coarser fractions is well below the acceptable limits, whereby the coarser fraction/s may be used in the manufacture of fertilizers and the fine fraction in the production of concrete, for instance. In other words, the bark boiler ash may be utilized in full without any need to dump it into landfill.

Secondly when studying further the process discussed in WO-A2-2011151511 in connection with a pulp mill it was learned that the microbe cultivation did not grow in the manner expected in the fermentation reactor (ethanol production) though the external circumstances were optimal. It was thus obvious that there was something wrong with the raw material fed into the fermentation reactor. Therefore, the constitution of the fibrous slurries was studied in more detail. It was learned that the fibrous slurries contain inorganic impurities, which interfere the operation of the microbes and prevents the efficient growth of the cultivation. As a consequence, in the present invention, the fibrous slurries were divided or separated into at least two fractions, for instance, by means of centrifugal separation, i.e. by means of hydrocyclone/s. The light mainly organic fraction of the separation was taken to the fermentation reactor whereafter the behavior, i.e. the growth of the microbe cultivation was as expected. The mainly inorganic coarse fraction is for the main part rejectable material that may be dumped as landfill. However, as the anaerobic biogas production or the algae farming are not sensitive to the constituents of the coarse mainly inorganic faction, it may be taken therein, too. Also, the coarse mainly inorganic fraction may be taken to the fertilizer production or to the production of the construction material.

The above discussed changes in the process compared to the prior art processes bring about several advantages.
- Nutrient (nitrogen, phosphorus, potassium) loading to air and water is considerably reduced,
- The nutrients are transformed to a soluble state that plants may use efficiently,
- The nutrients are recovered in the fertilizer circulation,
- The inventive concept offers the pulp and paper mill a new source of significant income,
- New products for pulp and paper industry, i.e. ethanol, bio gas, fertilizer, additives for concrete production,
- Protein, DHA (Docosahexaenoic acid, an omega-3 fatty acid), EPA (Eicosapentaenoic acid, another omega-3 fatty acid), etc. may be recovered by processing algae,
- Present day expenses caused by incinerating various slurries are saved,
- The amount of waste material taken to landfill is minimized, whereby the costs are also minimized, and
- The concept makes it possible to enter emission trading.

Figure 3 discusses schematically a way of solving, by means of the present invention, at least some of the problems found in the prior art pulp and/or paper mills. Here, the starting point is naturally the same, i.e. pulp and/or paper mill 52 discharges various waste flows shown for simplicity with one arrow 100. They may contain reject flows from various screening, filtering and washing devices, effluent flows from digestion, delignification and bleaching stages of a pulp mill, rejects and filtrates from paper machine short circulation, wire section, press section, broke handling, and effluents from chemical recovery etc. just to name a few examples, but by no means limiting the flows. Now, due to the possibilities given by the present invention either the filters, screens, hydrocyclones or any other filtering, classifying or sorting devices used for separating acceptable fiber material from the rejectable waste flows may be, but are not necessarily, run aiming at maximum fiber separation, or new separating devices 102 may be, but are not necessarily, arranged to treat the various flows so that the acceptable fiber material 104 is separated as efficiently as possible from the waste flow 54. In fact, the separating device 102 may be considered to represent the last separation stage or step of each particular waste flow at the paper or pulp mill, the separation stage or step being now run more efficiently than before as there is no need to allow acceptable fibrous material pass the separation in order to have more combustible material in the bark boiler. In other words, by means of the present invention the recovery of fiber material may be improved significantly either by running the existing separation devices more efficiently or by installing new separation devices in appropriate locations in the process. Thus the flow 54, which is now from the mill's point of view waste flow, may contain much less fibrous material than before.

The flow 54 is taken to an appropriate treatment device 56, which may be a prior art primary clarifier, though also other separation devices may be applied. The treatment device 56 divides the waste flow into a primary slurry 58 and a first effluent 60. In accordance with a preferred embodiment of the present invention the primary slurry 58 is taken to a further separator unit 98, which may be a single device or a series of devices, like hydrocyclones, coupled either in series and/or in parallel and in one or more stages or steps. The operation of the separator unit 98 may, in some cases be based, not only on centrifugal separation by means of hydrocyclones, but also on screening or filtration by means of pressure screens, curved screens, centrifugal separators or any combination thereof, just to name a few alternatives without any intention to limit the invention to the listed devices only. The separator unit 98 divides the primary slurry into one or more coarse fractions and a fine fraction. The fine fraction, i.e. the fraction containing mainly organic matter, is taken to a bio refinery 80, as it includes both fine and dissolved organic matter that may be used as "raw material" at the bio refinery, and especially in the production of ethanol if such a step is available at the bio refinery. The coarser fraction/s, which contain/s large-sized mainly inorganic impurities and possibly some knots and/or other invaluable organic particles may be either dumped, for instance to landfill, or utilized in the production of fertilizer at 90 and/or in the construction material industry at 86 in the production of concrete, for instance.

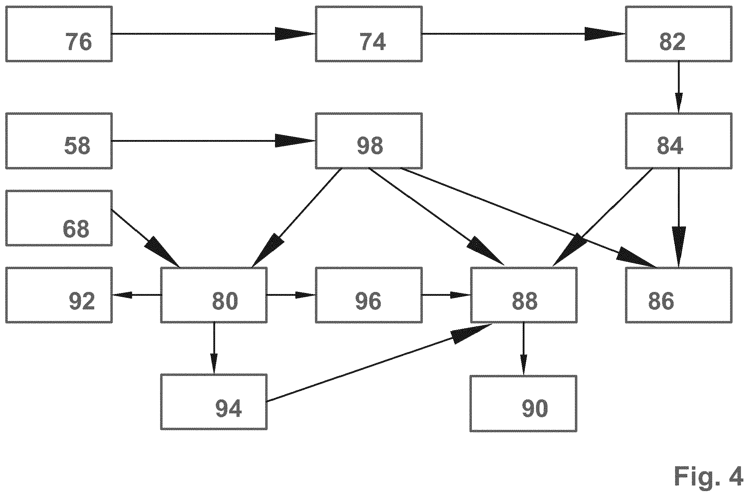

The first effluent 60 is taken to a secondary clarifier 62, which is normally a biological wastewater treatment facility. The secondary clarifier may also receive waste flows from the wood yard and/or from the coater of the paper machine. The secondary clarifier 62 produces a second slurry 68, and a clear effluent 66 that is discharged to rivers, lakes, seas or oceans, or taken back to use at the pulp and/or paper mill. The second slurry 68 so called bio slurry is taken to the biogas production in the bio refinery 80 the operation of which, as will be discussed in more detail in connection with Figure 4, results in the production of, for instance, a fertilizer 90. Now, as can be seen in Figure 3, the described process does not have any other waste flows to introduce to the bark boiler 74 than the hog fuel 76, i.e. the bark and other wood based combustible material from the wood yard. Naturally, the bark boiler may be provided with other fuels like peat or waste wood, i.e. wood that cannot for some reason be used for pulp, paper, tissue or board production. Also, it is possible to introduce in the bark boiler some material recovered from the waste flow 54, like for instance knots, but, on the one hand, the amount of such material is negligible compared to the overall amount of combustible material fed in the bark boiler, and on the other hand, the water content of such material may be easily minimized by pressing, for instance, whereby, in fact, such a material does not have any effect in the operation of the bark boiler. In this manner, the ash 82 recovered from the bark boiler 74 is in perfect condition in view of classifying the ash into fractions lean in and rich in heavy metals as well as to fractions applicable in then production of fertilizer and fractions applicable for use in, for instance, construction material industry.

Figure 4 discusses schematically treatment options for different side and waste flows of a pulp mill and/or a paper mill in accordance with the present invention. Of the pulp mill processes only the bark boiler 74 and the bio refinery 80 have been shown in Figure 4. The bio refinery 80 includes at least one anaerobic digester for biogas production, and/or at least one fermentation reactor for ethanol production, and, if so desired, at least one algae cultivation unit (algae farming). In other words, the bio refinery may be comprised of 1) an ethanol production, 2) an ethanol production and biogas production, 3) an ethanol production, biogas production and algae farming, 4) an ethanol production and algae farming, 5) biogas production, or 6) biogas production together with algae farming. Additionally, the bio refinery may include other treatment devices or units including but not limited to filters, thickeners, presses etc. In its most simple variation the present invention discusses the treatment of two raw materials, i.e. waste and side flows, in a bark boiler and in a bio refinery 80. The first raw material is bark and other material, i.e. hog fuel 76 originating from wood or logs at the wood yard, though also peat, waste wood and other appropriate fuels may be added thereto. The second raw material is formed of fibrous slurries 58 collected as residues from the various treatment stages of the pulp or paper mill, including, but not limited to, rejects from various screening, filtering and washing devices, effluent flows from digestion, delignification and bleaching stages of a pulp mill, effluents from chemical recovery, 0-water, paper mill filtrates etc.

In another variation of the present invention the bio refinery 80 uses as the third raw material, the bio slurries 68 originating from the biological waste water treatment plant of the pulp and/or paper mill.

In accordance with the present invention the bark and other wood material 76 originating mainly from the wood yard, i.e. the hog fuel, are incinerated in the bark boiler 74. Now that the heat generated by the bark boiler 74 may be used entirely for instance in steam generation, electricity generation, district heating etc. and not wasted a significant part thereof in evaporating water from fibrous and/or bio slurries, the efficiency ratio of the bark boiler is greatly improved. Now the ash 82 resulting from the combustion in the bark boiler contains heavy metals, but, as discussed already above, since the particle size distribution of ash 82 is very broad, and since the heavy metals, by nature, have adhered in combustion to the fine ash fraction, it is possible to divide the ash 82 at 84 in at least one fine ash fraction rich in heavy metals and in at least one coarse ash fraction lean in heavy metals. The fraction rich in heavy metals may, for instance, be taken to construction material industry 86 where the fine ash fraction may be used to replace part of the cement in concrete production. The fine ash fraction may, naturally, be used as landfill, too. The fraction/s lean in heavy metals may be taken to fertilizer production at 88. The separation stage 84 is performed, preferably but not necessarily, by means of a pneumatic separator.

A preferred way of classifying or fractionating the ash 82 at 84 is described in the following:
The ash to be fractionated or classified is proportioned carefully to a low pressure gas flow, the volume flow of which may be steplessly adjusted. The well adjusted ash-gas suspension is guided tangentially and with a chosen speed to a cylindrical separator device, in which a strong vortical flow is formed. Due to the vortical flow the larger ash particles collect against the inner surface of the separator device along which they flow downwards as the flow speed thereof is decreasing. The upper end of the cylindrical separator device is provided with a rotor whereby the fine fraction is able to escape between the vanes of the rotor along with the working gas. The rotational speed of the rotor may be adjusted and by means of its circumferential speed the heavier solid ash particles are prevented from entering among the fine fraction. The lower end of the separator device is conical. Slightly pressurized flushing air may be introduced to the bottom end of the cone for further washing fine particulate matter from the coarse product. The construction of the discharge conduit for the coarse matter is such that working gas or flushing air cannot escape via the conduit. In case too much fine fraction remains in the coarse fraction, the coarse fraction may be treated anew by means of a corresponding device, whereafter the fine fractions, containing most of the heavy metals, received from the two fractionating stages working in series may be combined and the coarse fraction of the second stage may be regarded as another coarse end product. The above discussed fractionation process may be arranged to operate in batch principle, in which the products are ready when their proportions are the ones desired in view of the recovered amount of fine fraction.

Coming back to the "raw materials" of the waste and side flow treatment facility, the fibrous slurry 58 is, in accordance with the present invention, taken to a separator unit 98 to be fractionated in at least a light mainly organic and a heavy mainly inorganic fraction, preferably by means of a hydrocyclone/centrifugal cleaner unit having one or more stages. The mainly organic light fraction containing, for instance dissolved organic material, fines etc. is taken to the bio refinery 80 and the heavy mainly inorganic fraction containing, for instance sand, larger knot fractions, etc. to the fertilizer production 88, or optionally to construction material industry 86, i.e. to the production of construction materials, like an additive for concrete production. Naturally the heavy fraction may also be used as landfill. The separation may also be performed such that three fractions are collected so that the lightest, mainly organic fraction goes to bio refinery 80, preferably to the fermentation reactor though also anaerobic digestion will work, and the two coarser mainly inorganic fractions to fertilizer manufacture 88 and the construction material industry 86. As to the separating or classifying the fibrous waste flow into two or more fractions in a separator unit 98, the invention covers all such separation stages treating the fibrous waste flow 58 that one of the flows, i.e. the lightest or finest mainly organic fraction is taken to the bio refinery, and another fraction/the other fractions either to the fertilizer production, construction material production, landfill or to any other position where the fraction is utilized. The coarser fraction/s may also be introduced to anaerobic digester and/or algae farming, if the bio refinery includes such, as they are not sensitive to the constituents of the coarse fraction/s. Also the separation unit 98may be any known apparatus that is able to perform its task in the desired manner.

In other words the bio refinery 80 of the present invention, depending on its variation, is able to use as its raw material, preferably, both bio slurries 68 and a selected part of the fibrous slurries 58, if each one of them is available, if not, the bio refinery has to manage with the raw material available.

In accordance with the simplest variation of the present invention the mainly organic fraction of the fibrous slurry 58 is taken to fermentation for the production of ethanol, and the digestate from the fermentation is taken to the manufacture of the fertilizer. In a further developed variation of the present invention the mainly organic fraction is taken to fermentation, ethanol is produced and the digestate is taken to anaerobic digestion for the production of biogas, whereafter the digestate of the anaerobic digestion is taken to fertilizer production. And in a further developed variation of the present invention the digestate of the fermentation or of the anaerobic digestion is filtered and the filtrate is taken to algae farming for growing algae such that algae mass may be utilized in the fermentation or in the anaerobic digestion. In the variations including the anaerobic digestion bio slurry 68 is taken therein for increasing the production of biogas.

Thus, in case the treatment of the waste flows takes place in both the fermentation and the anaerobic digestion and if both bio and fibrous slurries are available, at least ethanol and bio gas/methanol 92, nitrous compound 94, for instance in the form of nitrogen sulphite, and residual slurry 96, so called digestate may be recovered. However, as mentioned already above the bio refinery 80 may not have all the treatments steps, whereby, for instance, it is possible that the bio refinery 80 does not produce biogas/methane, i.e. the anaerobic digestion has not been arranged in the bio refinery. And consequently, since the nitrogen compound is normally stripped from biogas, there may not be any nitrogen compound that could be separated. However, the nitrogen originating from fibrous slurries 58 is present, has changed to a soluble state in fermentation and remains in the residual slurry 96 or digestate, recovered from the bio refinery 80, whereby it will end up in the fertilizer anyhow. Since the nitrogen is in soluble format when entering the fertilizer the plants are able to use it very efficiently. The same applies to phosphorus, which is transformed in fermentation or in anaerobic digestion in a soluble state.

The end products of the bio refinery of the present invention will be discussed in more detail in the following. The ethanol 82 and bio gas/methane/methanol 92 are valuable end products, which may be sold for further processing or combusted for creating electricity, for instance. The nitrous compound 94, if available, may be introduced to the production 88 of the fertilizer 90. The residual slurry 96, so called digestate is dewatered and then taken to be mixed with the ash fraction lean in heavy metals together with the nitrous compound 94. The heavy fraction, or one of the heavier fractions, from the separation/fractionation stage 98 of the fibrous slurries 58 may also be introduced to the fertilizer manufacture. An advantage of bringing the heavy/heavier mainly inorganic fraction from the separation stage 98 of fibrous slurries in the fertilizer manufacture is that the consistency of the mixture is easily so high that the further dewatering may be performed by means of a screw press. After dewatering the mixture is granulated and packed in sacks to be sold as a fertilizer. The resulting fertilizer may be organic or non-organic fertilizer depending on the origin of its raw materials.

As to the use of pulp mill waste flows in the present invention, the motivation of such will be discussed in more detail in the following. The flows originating from the fiberline (including, but not limited to, mechanical or chemical pulp production, delignification, bleaching, screening, filtering, washing stages, etc.) or chemical recovery of a pulp mill, stock preparation, wire section and/or press section of a paper, board or tissue machine have traditionally been taken towards waste water treatment or disposal already at such a stage that there is valuable recoverable material left in the flows. The flows may contain fiber material or filler material that could be recovered. These materials are here, and above, called fibrous slurries. However, as the prior art incineration of the bio slurry in the bark boiler has required that a certain amount of fibrous slurries is introduced to the bio slurry so that its dry matter content could be raised closer to combustible level, the required amount of fibrous slurries are wasted. In accordance with the present invention, the organic waste and side flows of the pulp and/or paper mill containing lignin, celluloses, hemicelluloses, etc. dissolved from the wood material in at least one of digestion delignification and bleaching, as well as fibre and filler residues etc. may be used as raw material in the bio refinery, i.e. in at least either the ethanol production, or in the anaerobic digester, or in both and possibly further boosting the process with the use of algae. In fact, it has been learned that the introduction of the dissolved organic material in the bio refinery, in place of for instance incinerating such in the recovery boiler or bark boiler, boosts significantly the operation of the biogas process. In practice, the waste and side flows may be utilized in full. An advantageous flow of a pulp mill that may be utilized by the method of the present invention is the filtrate collected from the washer or wash press located after the delignification stage at the pulp mill. The filtrate contains all the organic matter dissolved from pulp to the liquid phase in the numerous washing steps of the pulp mill bleaching and delignification stages. It should be understood that the water collected as delignification filtrate not only contains the organic matter and nutrients dissolved in the delignification but also those dissolved in at least a few bleaching stages, as the liquid has been brought to the delignification stage washer/wash press counter currently from the bleaching stages. The well proven prior art practice has been to introduce the filtrate of the delignification stage washer/wash press as washing liquid to the brown stock washer from where the filtrate has been taken to chemical recovery, whereby all the organic matter together with the nutrients in the filtrate has been combusted in the recovery boiler. Now, the present invention suggests a novel method of utilizing the dissolved organic matter and nutrients (phosphorus and nitrogen) in the bio refinery.

The above discussed treatment of various pulp and paper industry waste and side flows in a bio refinery to result in a residual slurry that contains various nutrients and compounds also improves the properties of the fertilizer so that the resulting fertilizer may be used not only as a fertilizer but also for replacing the use of potassium as the soil improving agent, just to name one further advantage. The nutrients, for instance phosphorus and nitrogen, recovered from the above discussed process are in soluble format, in the same manner as in chemically manufactured fertilizers, too, whereby the plants may easily and quickly utilize the nutrients. With regard to the inorganic matter originating from the fibrous slurries, i.e. the heavy fraction, it may be used as such in the production of concrete.

As an evidence of the advantageous properties of the fertilizer produced in accordance with the present invention the results of a test are hereby presented.

Bio slurry received from a waste water treatment facility of a pulp mill was taken to an anaerobic digester for the production of bio gas, the digestate from the anaerobic digester was taken to the manufacture of a fertilizer to which also fractionated ash from a bark boiler was introduced. The ash from a bark boiler combusting mere hog fuel was fractionated such that the heavy metals were concentrated in the fine fraction whereas the coarse fraction was taken to the fertilizer production. The fertilizer was manufactured as discussed in the above specification and was given to be examined and tested by the University of Helsinki. The test results showed that the fertilizer fulfilled the requirements of an organic fertilizer and that the fertilizer gave 10 % higher or even better growth than commercially available chemical fertilizers.

One thing worth mentioning is the adjusting of the consistency of the various flows and slurries in the above discussed process. Mostly because the pulp and paper mills use devices, which differ a great deal from each other, for screening, filtering or dewatering various flows, also the consistencies of the resulting flows are variable. The varying consistencies of both flows entering to the process of the invention and the flows circulating between various process stages in the process of the present invention may require dewatering or dilution of the flows. Since the different dilution or dewatering steps do not change the basic features or principles of the invention, they have not been discussed in any more detail above.

It is to be noted that above only a few most preferred embodiments or variations of the present invention have been discussed. Thus, it is obvious that the invention is not restricted to the above described embodiments and variations, but it may be applied in many different ways within the scope of the appended claims.

## Claims

1. A method of processing waste and side flows of pulp and paper industry, the waste and side flows comprising at least bark and other wood based waste flows, so called hog fuel (76), and at least one fibrous waste flow (54); the processing arrangement of a pulp and/or a paper mill (52) comprising at least a bark boiler (74) for combusting hog fuel, a biological wastewater treatment plant (62) and a bio refinery (80) comprising at least fermentation/ethanol production, the method comprising the steps of introducing hog fuel (76) to the bark boiler (74) for producing ash (82), introducing the at least one fibrous waste flow (54) to a treatment device (56) for dividing the at least one fibrous waste flow (54) to a fibrous primary slurry (58) and a first effluent (60), and introducing the first effluent (60) to the biological wastewater treatment plant (62), the method being
**characterized by** the steps of
a) Providing the processing arrangement with a separator unit (98),
b) Introducing the fibrous primary slurry (58) into the separator unit (98),
c) Dividing the fibrous primary slurry by means of the separator unit (98) into at least two fractions, i.e. a light mainly organic fraction and at least one heavy mainly inorganic fraction,
d) Introducing the light mainly organic fraction to the bio refinery (80) to fermentation for the production of ethanol and/or biogas and digestate (96),
e) Taking the digestate (96) from the bio refinery (80) to fertilizer production (88),
f) Taking the ash (82) from the bark boiler (74) to fractionation (84),
g) Fractionating the ash into at least one coarse fraction lean in heavy metals and a fine ash fraction rich in heavy metals,
h) Taking the at least one coarse ash fraction lean in heavy metals to fertilizer production (88) for producing a fertilizer mixture together with the digestate.

2. The method as recited in claim 1, **characterized by** providing the biorefinery with anaerobic digestion/biogas production.

3. The method as recited in claim 1 or 2, **characterized by**, prior to step e) introducing at least one bio slurry (68) in the bio refinery (80).

4. The method as recited in claim 1, 2 or 3, **characterized by** dewatering the digestate (96) prior to combining such with the at least one coarse ash fraction.

5. The method as recited in claim 3, **characterized by** treating the light mainly organic fraction and the at least one bio slurry (68) in the bio refinery (80) to produce a nitrous compound (94).

6. The method as recited in claim 5, **characterized by** introducing the nitrous compound (94) to fertilizer production (88).

7. The method as recited in claim 6, **characterized by** dewatering the fertilizer mixture prior to combining such with the nitrous compound (94).

8. The method as recited in claim 1, **characterized by** dewatering the fertilizer mixture prior to granulating and packaging.

9. The method as recited in any one of the preceding claims, **characterized by** introducing organic material dissolved in at least one of digestion, delignification and bleaching to the bio refinery (80).

10. The method as recited in claim 2 and 9, **characterized by** using the dissolved organic material for boosting the operation of the biogas production.

11. The method as recited in claim 1, **characterized by** using the fine ash fraction rich in heavy metals and/or at least one heavy mainly inorganic fraction as an additive in construction material industry.

12. The method as recited in claim 1, **characterized by** collecting the fibrous waste flows from mechanical, chemi-mechanical and/or chemical pulp production and/or, paper, board and/or tissue production like at least one of reject flows of chip screening, brown stock screening, fine screening, centrifugal cleaning, filtrate waste flows from filtering/washing stages in connection with digestion, brown stock washing, delignification, bleaching, various reject and filtrate flows from paper machine short circulation, wire section, press section, broke handling, and effluents from chemical recovery.

13. The method as recited in claim 1, **characterized in that** the separator unit (98) is at least one hydrocyclone.

14. A fertilizer produced in accordance with the method of any one of the preceding claims, **characterized in that** the fertilizer (90) is formed, at least partially, of the digestate (96) collected from a bio refinery treating one or more fibrous waste flows of a pulp and/or paper mill from which fibrous waste flows heavy mainly inorganic fraction is separated and of an ash fraction lean in heavy metals collected from a bark boiler of a pulp and/or paper mill.

15. The fertilizer as recited in claim 14, **characterized in that** the fertilizer (90) further comprises nutrients, like nitrogen and phosphorus, collected from the biorefinery in soluble format.

16. The fertilizer as recited in claim 14 or 15, **characterized in that** the fertilizer (90) comprises potassium collected from the biorefinery, whereby the fertilizer may also be used as a soil improving agent.

## Patentansprüche

1. Verfahren zur Bearbeitung von Abfall- und Nebenströmen der Zellstoff- und Papierindustrie, wobei die Abfall- und Nebenströme mindestens Baumrinde und andere holzbasierte Abfallströme, sogenannten Abfallbrennstoff (76), und mindestens einen faserhaltigen Abfallstrom (54) umfassen; wobei die Verarbeitungsanordnung einer Zellstoff- und/oder Papierfabrik (52) mindestens einen Rindenkessel (74) zum Verbrennen des Abfallbrennstoffes, eine biologische Abwasserbehandlungsanlage (62) und eine Bioraffinerie (80) umfasst, die mindestens eine Fermentations/Ethanolproduktion umfasst, wobei das Verfahren folgende Schritte umfasst: Einführen des Abfallbrennstoffs (76) in den Rindenkessel (74), um Asche (82) zu produzieren, Einführen des mindestens einen faserhaltigen Abfallstroms (54) in eine Behandlungsvorrichtung (56), um den mindestens einen faserhaltigen Abfallstrom (54) in eine faserhaltige primäre Aufschlämmung (58) und ein erstes Abwasser (60) zu teilen, und Einführen des ersten Abwassers (60) in die biologische Abwasserbehandlungsanlage (62), wobei das Verfahren durch die folgenden Schritte gekennzeichnet ist:
a) Bereitstellen der Verarbeitungsanordnung mit einer Abscheideeinheit (98),
b) Einführen der faserhaltigen, primären Aufschlämmung (58) in die Abscheideeinheit (98),
c) Aufteilen der faserhaltigen, primären Aufschlämmung mithilfe der Abscheideeinheit (98) in mindestens zwei Fraktionen, d. h. eine leichte, hauptsächlich organische Fraktion und mindestens eine schwere, hauptsächlich anorganische Fraktion,
d) Einführen der leichten, hauptsächlich organischen Fraktion in die Bioraffinerie (80) zur Fermentation für die Produktion von Ethanol und/oder Biogas und Gärrest (96),
e) Überführen des Gärrests (96) aus der Bioraffinerie (80) zur Düngerproduktion (88),
f) Überführen der Asche (82) aus dem Rindenkessel (74) zur Fraktionierung (84),
g) Fraktionieren der Asche in mindestens eine arm an Schwermetallen grobe Fraktion und eine reich an Schwermetallen feine Aschefraktion,
h) Überführen der mindestens einen arm an Schwermetallen groben Aschefraktion in die Düngerproduktion (88) zur Herstellung einer Düngermischung zusammen mit dem Gärrest.

2. Verfahren nach Anspruch 1, **gekennzeichnet durch** Bereitstellen der Bioraffinerie mit anärober Gärung/Biogasproduktion.

3. Verfahren nach Anspruch 1 oder 2, **gekennzeichnet durch** Einführen von mindestens einer Bioschlämme (68) in die Bioraffinerie (80) vor Schritt e).

4. Verfahren nach Anspruch 1, 2 oder 3, **gekennzeichnet durch** Entwässern des Gärrests (96) vor dessen Kombination mit der mindestens einen groben Aschefraktion.

5. Verfahren nach Anspruch 3, **gekennzeichnet durch** Behandeln der leichten, hauptsächlich organischen Fraktion und der mindestens einen Bioaufschlämmung (68) in der Bioraffinerie (80), um eine salpeterhaltige Verbindung (94) zu erzeugen.

6. Verfahren nach Anspruch 5, **gekennzeichnet durch** Einführen der salpeterhaltigen Verbindung (94) in die Düngerproduktion (88).

7. Verfahren nach Anspruch 6, **gekennzeichnet durch** Entwässern der Düngermischung vor ihrer Kombination mit der salpeterhaltigen Verbindung (94).

8. Verfahren nach Anspruch 1, **gekennzeichnet durch** Entwässern der Düngermischung vor Granulieren und Verpacken.

9. Verfahren nach einem der vorhergehenden Ansprüche, **gekennzeichnet durch** Einführen von organischem Material, das in mindestens einem von Gärung, Delignifizierung und Bleichen aufgelöst wurde, in die Bioraffinerie (80).

10. Verfahren nach Anspruch 2 und 9, **gekennzeichnet durch** Anwenden des aufgelösten, organischen Materials, um den Betrieb der Biogasproduktion zu steigern.

11. Verfahren nach Anspruch 1, **gekennzeichnet durch** Anwenden der reich an Schwermetallen feinen Aschefraktion und/oder der mindestens einen schweren, hauptsächlich anorganischen Fraktion als ein Zusatzstoff in der Baumaterialindustrie.

12. Verfahren nach Anspruch 1, **gekennzeichnet durch** Sammeln der faserhaltigen Abfallströme aus mechanischer, chemi-mechanischer und/oder chemischer Zellstoffproduktion und/oder Papier-, Pappe- und/oder Tissueproduktion wie mindestens einem von Ausschussströmen von Hackschnitzelsortierung, Braunstoffsieben, Feinsieben, Zentrifugalreinigen, Filtratabfallströmen aus Filtrier/Waschstufen in Verbindung mit Gärung, Braunstoffwaschen, Delignifizierung, Bleichen, verschiedenen Ausschuss- und Filtratströmen aus kurzem Papiermaschinenumlauf, Siebpartie, Pressenpartie, Ausschussverarbeitung und Abwässern aus chemischer Rückgewinnung.

13. Verfahren nach Anspruch 1, **dadurch gekennzeichnet, dass** die Abscheideeinheit (98) mindestens ein Hydrozyklon ist.

14. Ein nach dem Verfahren von einem der vorstehenden Ansprüche hergestellter Dünger, **dadurch gekennzeichnet, dass** der Dünger (90) mindestens teilweise aus dem Gärrest (96) gebildet wird, der aus einer Bioraffinerie gesammelt wird, die einen oder mehrere faserhaltige Abfallströme einer Zellstoff- und/oder Papierfabrik behandelt, von denen die schwere, hauptsächlich anorganische Fraktion der faserhaltigen Abfallströme abgetrennt wird; sowie aus einer arm an Schwermetallen Aschefraktion, die aus einem Rindenkessel einer Zellstoff- und/oder Papierfabrik gesammelt wird.

15. Dünger nach Anspruch 14, **dadurch gekennzeichnet, dass** der Dünger (90) ferner Nährstoffe wie Stickstoff und Phosphor enthält, die aus der Bioraffinerie in löslicher Form gesammelt werden.

16. Dünger nach Anspruch 14 oder 15, **dadurch gekennzeichnet, dass** der Dünger (90) Kalium enthält, der aus der Bioraffinerie gesammelt wird, wodurch der Dünger ebenfalls als ein Mittel zur Verbesserung der Bodenqualität verwendet werden kann.

## Revendications

1. Procédé de traitement de flux de déchets et de matières recyclables issus de l'industrie papetière, les flux de déchets et de matières recyclables comprenant au moins de l'écorce ainsi que d'autres flux de déchets à base de bois, ainsi appelés déchets de bois (76), et au moins un flux de déchets fibreux (54) ; le dispositif de traitement d'une usine de pâte à papier et/ou de papier (52) comprenant au moins une chaudière à écorces (74) permettant la combustion des déchets de bois, une usine de traitement des eaux usées biologiques (62) et une bioraffinerie (80) comprenant au moins la fermentation/production d'éthanol, le procédé comprenant les étapes d'introduction de déchets de bois (76) dans la chaudière à écorces (74) permettant la production de cendres (82), l'introduction de l'au moins un flux de déchets fibreux (54) dans un dispositif de traitement (56) permettant de diviser l'au moins un flux de déchets fibreux (54) en un coulis primaire fibreux (58) et en un premier effluent (60), et l'introduction du premier effluent (60) dans l'usine de traitement des ou usés biologiques (62), le procédé étant **caractérisé par** les étapes de
a) fourniture du dispositif de traitement muni d'une unité séparatrice (98),
b) introduction du coulis primaire fibre (58) dans l'unité séparatrice (98),
c) la division du coulis primaire fibreux au moyen de l'unité séparatrice (98) dans haut moins de fractions, c.-à-d. une fraction légère principalement organique et au moins une fraction lourde principalement inorganique,
d) introduction de la fraction légère principalement organique dans la bioraffinerie (80) à la fermentation pour la production d'éthanol et/ou de biogaz et de digestat (96),
e) envoi du digestat (96) provenant de la bioraffinerie (80) vers la production d'engrais (88),
f) envoi des cendres (82) provenant de la chaudière à écorces (74) vers leur fractionnement (84),
g) fractionnement des cendres en au moins une fraction grossière pauvre en métaux lourds et d'une fraction de cendres fines riche en métaux lourds,
h) envoi de l'au moins une fraction de cendres grossières pauvre en métaux lourds vers la production d'engrais (88) permettant la production d'un mélange d'engrais conjointement au digestat.

2. Procédé selon la revendication 1, **caractérisé par** la fourniture de la bioraffinerie équipée d'une digestion aérobique/production de biogaz.

3. Procédé selon la revendication 1 ou la revendication 2, **caractérisé par**, avant l'étape e), l'introduction d'au moins un coulis biologique (68) dans la bioraffinerie (80).

4. Procédé selon la revendication 1, 2 ou 3, **caractérisé par** l'assèchement du digestat (96) avant sa combinaison avec l'au moins une fraction de cendres grossières.

5. Procédé selon la revendication 3, **caractérisé par** le traitement de la fraction légère principalement organique et de l'au moins un coulis biologique (68) dans la bioraffinerie (80) pour produire un composé nitré (94).

6. Procédé selon la revendication 5, **caractérisé par** l'introduction du composé nitré (94) dans la production d'engrais (88).

7. Procédé selon la revendication 6, **caractérisé par** l'assèchement du mélange d'engrais avant sa combinaison avec le composé nitré (94).

8. Procédé selon la revendication 1, **caractérisé par** l'assèchement du mélange d'engrais avant la granulation et le conditionnement.

9. Procédé selon l'une quelconque des revendications précédentes, **caractérisé par** l'introduction de matières organiques dissoutes dans au moins un parmi la digestion, la délignification et le blanchissement dans la bioraffinerie (80).

10. Procédé selon la revendication 2 et la revendication 9, **caractérisé par** l'utilisation de la matière organique dissoute pour stimuler le fonctionnement de la production de biogaz.

11. Procédé selon la revendication 1, **caractérisé par** l'utilisation de la fraction de cendres fines riche en métaux lourds et/ou de l'au moins une fraction lourde principalement inorganique en tant qu'additif dans l'industrie des matériaux de construction.

12. Procédé selon la revendication 1, **caractérisé par** la collecte des flux de déchets fibreux à partir de production mécanique, chimio-mécanique et/ou chimique de pâte à papier et/ou, de production de papier, de carton et/ou de tissu comme au moins un parmi des flux de rejets du classage des copeaux, du classage de la pâte brune, du classage fin, du nettoyage centrifuge, des flux de déchets de filtrat à partir des étapes de filtration/lavage en connexion avec la digestion, du lavage de la pâte brune, de la délignification, du blanchissement, des divers flux de rejet et de filtrat à partir de la circulation courte de machine à papier, de la section toile, de la section des presses, de la manipulation des cassés et des effluents provenant de la récupération chimique.

13. Procédé selon la revendication 1, **caractérisé en ce que** l'unité séparatrice (98) est au moins un hydrocyclone.

14. Engrais produit conformément au procédé selon l'une quelconque des revendications précédentes, **caractérisé en ce que** l'engrais (90) est formé, au moins partiellement, du digestat (96) recueilli auprès d'une bioraffinerie traitant un ou plusieurs flux de déchets fibreux d'une usine de pâte à papier et/ou de papier à partir desquels une fraction lourde principalement inorganique de flux de déchets fibreux est séparée et d'une fraction de cendres pauvre en métaux lourds recueillie auprès d'une chaudière à cendres d'une usine de pâte à papier et/ou de papier.

15. Engrais selon la revendication 14, **caractérisé en ce que** l'engrais (90) comprend en outre des nutriments, tels que de l'azote et du phosphore, recueillis auprès de la bioraffinerie dans un format soluble.

16. Engrais selon la revendication 14 ou la revendication 15, **caractérisé en ce que** l'engrais (90) comprend du potassium recueilli auprès de la bioraffinerie, selon lequel l'engrais peut également être utilisé en tant qu'agent d'amélioration du sol.
